# EUROPEAN PATENT SPECIFICATION

(11) **EP 2 629 805 B1**
(45) Date of publication and mention of the grant of the patent: **20.05.2015**
(21) Application number: 11776494.4
(22) Date of filing: 19.10.2011
(51) Int. Cl.: A61L 2/10, A61B 5/022

(54) **CLEANING DEVICE**
REINIGUNGSVORRICHTUNG
DISPOSITIF DE NETTOYAGE

(30) Priority: 16.05.2011 GB 201108158; 19.10.2010 GB 201017573
(43) Date of publication of application: 28.08.2013
(73) Proprietor: Mantra Medical Limited, Sheffield, South Yorkshire S3 7SJ (GB)
(72) Inventor: HERCOCK, Paul Martin, Sheffield South Yorkshire S3 7SJ (GB); FREWER, Neil, Sheffield South Yorkshire S60 5WF (GB); JONES, Richard Anthony Barritt, Sheffield South Yorkshire S1 4SA (GB)
(74) Representative: Foot, Paul Matthew James
(86) International application number: PCT/GB2011/052016
(87) International publication number: WO 2012/052754

(56) References cited:
- WO-A1-2005/110001
- KR-A- 20080 083 899
- US-A1- 2002 162 972
- US-A1- 2008 230 099

## Description

The present invention relates to a cleaning device. More particularly, but not exclusively, the present invention relates to a device for cleaning a sphygmomanometer (blood pressure monitor) cuff by use of ultra violet (UV) light.

### Background to the invention

The sphygmomanometer is used by virtually all healthcare professionals in a huge range of circumstances. It typically consists of a pressure-measuring device (either electronic or analogue) connected by a piece of rubber tubing to a cuff designed to wrap around the patient's upper arm. The cuff is available in a variety of sizes from those needed for premature neonatal babies through to those designed for use with obese adults. The cuff (the part that comes into contact with the patient) is often used on numerous successive patients without being cleaned and studies have shown that the cuff of the sphygmomanometer is frequently colonised with large numbers of harmful bacteria (including MRSA and *C.difficile*), viruses and fungi. Accordingly the cuff may act as a source of transmission of potentially serious infections between patients causing significant morbidity and mortality.

Presently the accepted method for cleaning the cuff between patients is by using hand-held cleaning swabs, often alcohol-soaked. Unfortunately this method is not only time consuming and cumbersome but is also of questionable effectiveness, particularly because a significant portion of the surface of the cuff consists of a hook and loop fastening and such swabs are unable to effectively clean into the substance of the hooks and loops. In practice this often means that the cuff is simply not cleaned at all. WO2005/110001A1 (Rai), US2002/162972 (Pleet) and US 2008/230099 (Perlman) show various cleaning devices for medical equipment.

It is therefore desirable for healthcare professionals to be provided with an expedient and effective way to clean sphygmomanometer cuffs immediately before and after use to prevent or inhibit the spread of infection between patients by sphygmomanometer cuff.

It would be particularly desirable if such a method was available in the same place as the sphygmomanometer itself, preferably at the bedside, and it is important that the device affords rapid, effective and reliable disinfection. In addition, any device performing such a function should not itself become a source of infection, and being intended for use in a public health environment, ease of maintenance and safety are of importance.

The present invention seeks to overcome, or at least mitigate the problems of the prior art.

### Summary of the invention

To overcome this, the present invention proposes a device that will effectively and rapidly decontaminate the surfaces of a sphygmomanometer cuff, including within the hook and loop portion. Accordingly, one aspect of the present invention provides a device to clean and/or disinfect either or both surfaces of a sphygmomanometer cuff, the device comprising: an entrance through which a sphygmomanometer cuff may be introduced into a passageway; an exit from the passageway; a drive mechanism to feed a cuff through the passageway; and a source of cleaning and/or disinfection located so as to act on a sphygmomanometer cuff located within the passageway, wherein the passageway comprises a curved portion.

Preferably the device is configured to clean and/or disinfect both surfaces of a sphygmomanometer cuff simultaneously, more preferably the device comprises a source of cleaning and/or disinfection on first and second sides of the passageway to clean and/or disinfect both surfaces of a sphygmomanometer cuff simultaneously. The device of the present invention may rapidly and reliably disinfect both surfaces of a sphygmomanometer cuff.

The device is advantageously arranged in normal usage such that the entrance is above the exit. Preferably, the curved portion is provided proximate the exit.

The curved portion advantageously diverts the passageway through an angle of at least 30°.

The passageway may comprise a substantially straight portion housing the source of cleaning and/or disinfection.

Advantageously, the source of cleaning and/or disinfection is ultraviolet light. Ultraviolet is a well established method of disinfection that effectively kills viral, bacterial and fungal particles, rendering them non-infective.

The device preferably comprises a scattering arrangement to scatter the light and reach maximal coverage of a sphygmomanometer cuff surface. The scattering arrangement more preferably comprises a multi-faceted mirror.

In an alternative arrangement the source of cleaning and/or disinfection is a sonic source.

The drive mechanism is advantageously a gripping mechanism to engage an edge of the cuff in one preferred embodiment, or is additionally or alternatively a at least one feed roller.

In a preferred embodiment the feed roller is flexible and/or resiliently mounted to accommodate uneven thicknesses of sphygmomanometer cuffs.

The device may also advantageously comprise an adjustment mechanism for the passageway to permit the passageway to adjust to accommodate sphygmomanometer cuffs of differing overall thickness.

The device preferably further comprises a control mechanism to detect the presence of a sphygmomanometer cuff within the passageway, and only permit the source of cleaning and/or disinfection to be activated with a sphygmomanometer cuff present within the passageway.

Advantageously, the device further has a breakaway mechanism to enable the passageway to open in the event of an accidental inflation of a sphygmomanometer cuff within the device.

Alternatively, the device may further comprise an ejection mechanism to eject a sphygmomanometer cuff in the event of an accidental inflation of a sphygmomanometer cuff within the device so as to minimise the risk of damage occurring.

In a further alternative, the device may comprise a mechanism configured to mimic an artery and behaving in a manner in which blood pressure has been exceeded, so the cuff is caused to deflate so as to minimise the risk of damage occurring.

The device may conveniently comprise an attachment for mounting it on the 'obs machine' trolleys often used in hospitals to mount sphygmomanometers and hence may be mounted in close proximity to the sphygmomanometer itself for portability to the patient's bedside.

The device may further comprise an arrangement at at least one of the entry and exit to inhibit the escape of UV light therefrom. The arrangement preferably comprises at least one pair of opposed flexible fins. Even more preferably, a second pair and yet more preferably a third pair of fins having tips thereof offset from the first are provided.

The device preferably further comprises a track through which an inflation tube of a sphygmomanometer may extend external of the device and be guided as the cuff is cleaned.

The device advantageously further comprises a hand-held cleaning unit removable from the housing and configured to clean further portions of a sphygmomanometer, such as a cord thereof.

The device may advantageously further include a means of indicating when servicing or cleaning is required.

The device may further comprise a reader to read a unique identifier associated with a cuff to be cleaned and a controller linked to the reader to determine a function of the device based on information associated with the uniquely identified cuff.

A second aspect of the present invention provides a method of cleaning or disinfecting a sphygmomanometer cuff utilising a device as described above, comprising a step of feeding the sphygmomanometer cuff through the opening of the device into the passageway.

A specifically designed disposable maintenance swab, pad or device may be provided, which is passed through the sphygmomanometer cuff cleaning device to remove any residue accumulating within the device, thereby preventing the device becoming itself a source of infection.

The sphygmomanometer cuff may be left attached to the inflation tube extending from the pressure-measuring device/monitor during cleaning or, depending upon the embodiment of the device, the device may require that the cuff is disconnected from the pressure-measuring device, provide a means of automatically disconnecting the cuff, or a means of rejecting a cuff from the device that is still connected.

The device may be suitably dimensioned for use with any and all of the commonly used sizes of sphygmomanometer cuff.

It may incorporate safety features to prevent the ultraviolet light presenting a danger to healthcare workers, patients or their relatives.

The device of the present invention advantageously employs features designed to prevent the device itself from becoming a source of infection and is easy to clean.

Another aspect of the present invention provides a method of cleaning or disinfecting a sphygmomanometer cuff utilising a device of the preceding paragraphs, comprising a step of feeding the sphygmomanometer cuff through the opening of the device into the passageway.

In an example, a system for monitoring cleaning and/or disinfection of reusable healthcare equipment, such as sphygmomanometer cuffs, is provided the system comprising: a machine readable unique identifier associated with an item of reusable healthcare equipment; a reader configured to read the unique identifier associated with a cleaning device for cleaning and/or disinfecting the equipment; and a memory to persistently log cleaning events of the healthcare equipment.

The system preferably a further comprises a controller associated with the cleaning device, the controller being linked to the reader to determine a function of the device based on information associated with the uniquely identified item of healthcare equipment.

Advantageously, the device further comprises a transmitter to transmit data relating to cleaning events to a remote location.

Optionally, multiple devices are provided and the memory logs cleaning events at each device.

The memory may be associated with the healthcare equipment or the memory is at a remote location.

The system may further comprise a remote access device for accessing cleaning event information of the healthcare equipment. The remote access device may be a networked computer and the information may be supplied via a web page on an internet browser.

The function of the device may be a deep or standard clean function and/or whether to accept an item of healthcare equipment for cleaning.

### Brief description of the drawings

A device of the present invention will now be described by way of example only, with reference to the accompanying drawings, in which:-
Fig. 1 is a perspective view of the device of one embodiment of the present invention;
Fig. 2 is a front view of the device of Figure 1 and illustrates the pathway followed by the sphygmomanometer cuff as it moves through the device;
Fig 3 is a schematic view of a control system of the device of Figure 1;
Fig. 4 is an isometric view of a device according to a second embodiment of the present invention;
Fig. 5 is frontal view of the device of Fig. 4;
Fig 6 is a side elevation of the device of Fig 4, which schematically illustrates the layout of certain interior components;
Fig. 7 is a plan view of the device of Fig 4;
Fig. 8 is a side elevation of a device according to a third embodiment of the present invention; and
Fig. 9 is a side elevation of a device a system according to a fourth embodiment of the present invention for monitoring usage of devices, such as those of the first three embodiments.

### Detailed description of preferred embodiments

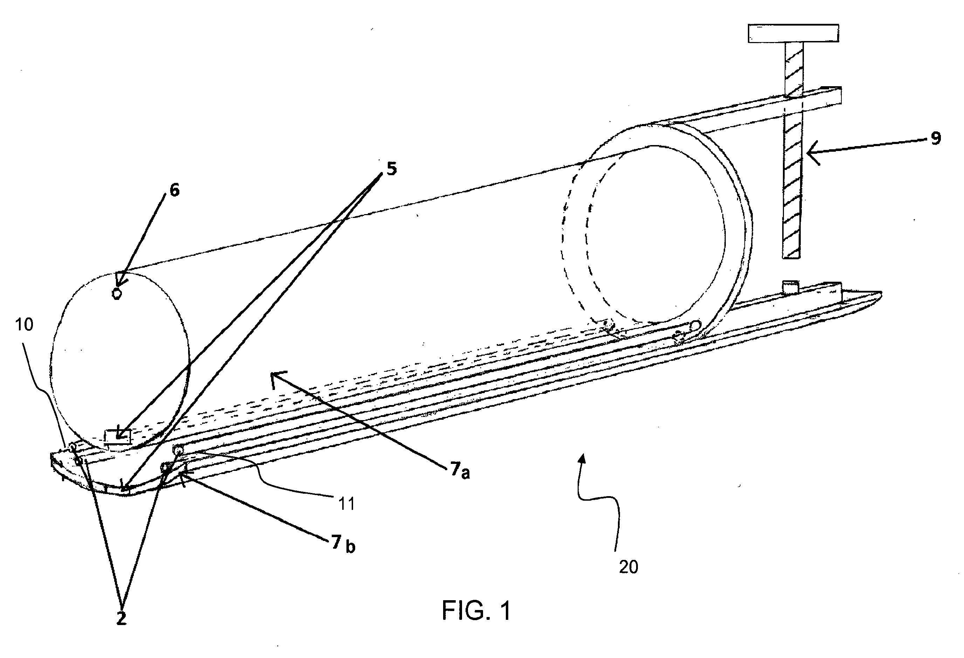

With reference to Figure 1, a device according to an embodiment of the present invention indicated generally at 20 comprises a major housing part 7a and a minor housing part 7b. The major part 7a is generally tubular in shape.

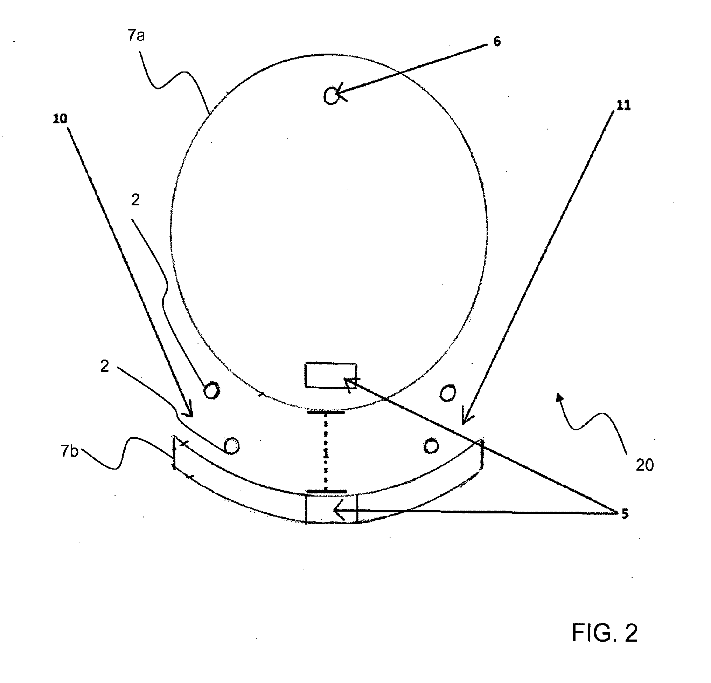

The minor part 7b is elongate, curved and has a radius of curvature centred on the same axis as the major part 7a. Thus, there is a substantially constant curved spacing between the major and minor housing parts to define a passageway 1 (see Fig. 2) between the two. The passageway 1 has two radial openings, an entrance 10 and an exit 11, and also has an end opening 24.

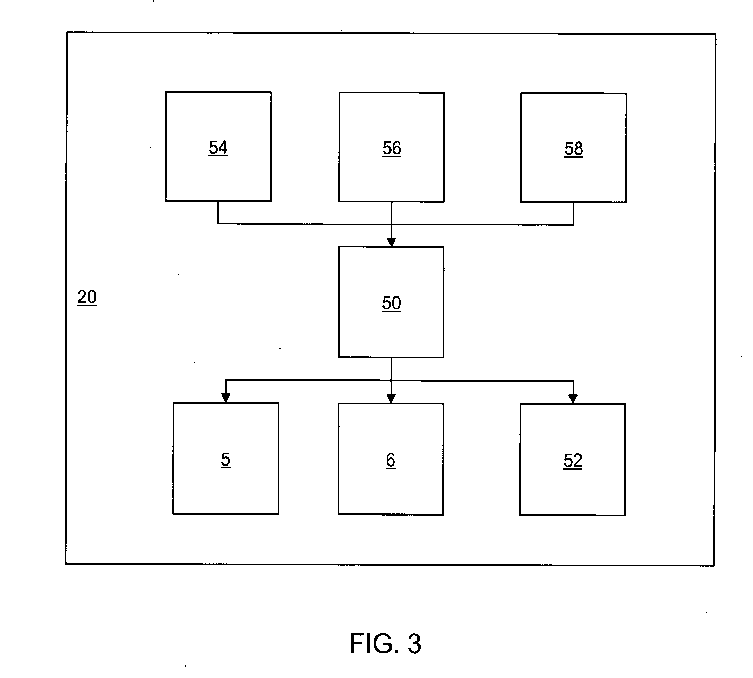

A pair of rollers 2 is provided at the entrance 10 and exit 11 along the length thereof. One or both of each pair of rollers 2 may be flexible so as to accommodate variations in the thickness of sphygmomanometer cuffs as they are fed through the device. In addition, the rollers 2 may advantageously be formed from a high friction material, such as artificial rubber in order to aid the feed of sphygmomanometer cuffs. In some embodiments (not shown), supports may be provided proximate the end opening to support the device against the major and minor housing parts 7a and 7b and the spacing of the major and minor parts may increase overall, e.g. by allowing the major and minor parts to separate. The rollers are driven by suitable drive means, such as electric motor(s) (52, Figure 3).

The major and minor housing parts 7a and 7b are each further provided with ultra violet lamps 5. The lamps are preferably cold cathode (CCFL) tube emitting 254 nm wavelength light as these are rapid to start and do not emit significant heat by comparison with other UV sources. Each lamp extends substantially the full length of the housing, and is located facing downwardly in the lower part of the major housing 7a, and facing upwardly from the minor housing 7b respectively, such that when a sphygmomanometer cuff is fed through the device 20 UV light is incident on both faces. To further ensure complete coverage of the sphygmomanometer cuff by UV light, the major and minor housings 7a and 7b are preferably further provided with a light scattering arrangement, e.g. a multi-faceted mirror (not shown) located behind each lamp.

In order to make the device 20 more convenient to use, it is further provided, in this embodiment, with a mounting arrangement in the form of a clamp 9 extending from the rear of the device. This is provided with the intention that the device may be mounted to an observation trolley upon which the sphygmomanometer is typically stored, meaning the device is close at hand.

In a preferred variant, the device may be provided with a breakaway mechanism (not shown) that is operable between the major and minor housing parts 7a and 7b so that if inflation of the sphygmomanometer cuff accidentally occurs whilst it is in the device 20, the minor part 7b will deflect (e.g. pivot or slide) relative to the major part 7a once a predetermined separating force is achieved. As a result, damage to device 20 or the sphygmomanometer is prevented. The mechanism may be reset once the sphygmomanometer cuff has been removed. In other embodiments (not shown), an alternative means may be provided to prevent damage to the device or a cuff, if a cuff is accidentally inflated within the device. In one embodiment, an inflation sensor and ejection mechanism may be provided. In another embodiment a mechanism may be provided within the device to mimic a patient's artery, and by behaving in a manner in which blood pressure has been exceeded, cause the cuff to deflate thereby avoiding damage occurring.

In a further preferred variant an upwardly extending lip (not shown) may be provided on the end of the minor part 7b or a downwardly extending lip on the end of the major part 7a to enable the non-cuff portions (inflation mechanism, tubing, display etc.) of the sphygmomanometer to continue to be positioned outside the device 20 during cleaning, but to act as a barrier to the escape of UV light from the device.

The device 20 preferably further comprises a control system illustrated schematically in Figure 3 to ensure safe and effective operation of the device. The control system is, in this embodiment, physically located within the major housing 7a, along with the motors 52, transformer and/or batteries (if the device is rechargeable).

The control system comprises a controller 50 (preferably a microprocessor) that receives input signals from an on/off switch 54 mounted on the exterior of the device 20, sensors 56 positioned to detect the presence of a sphygmomanometer cuff at the entrance 10 and exit 11 to the passageway (e.g. by pressure, light etc.) and a breakaway sensor 58 to sense if the breakaway mechanism has been activated.

The controller may emit output control signals to the UV lamps 5 to switch them on and off, an LED 6 mounted on the exterior of the device to indicate it is in use, and to the electric motors 52.

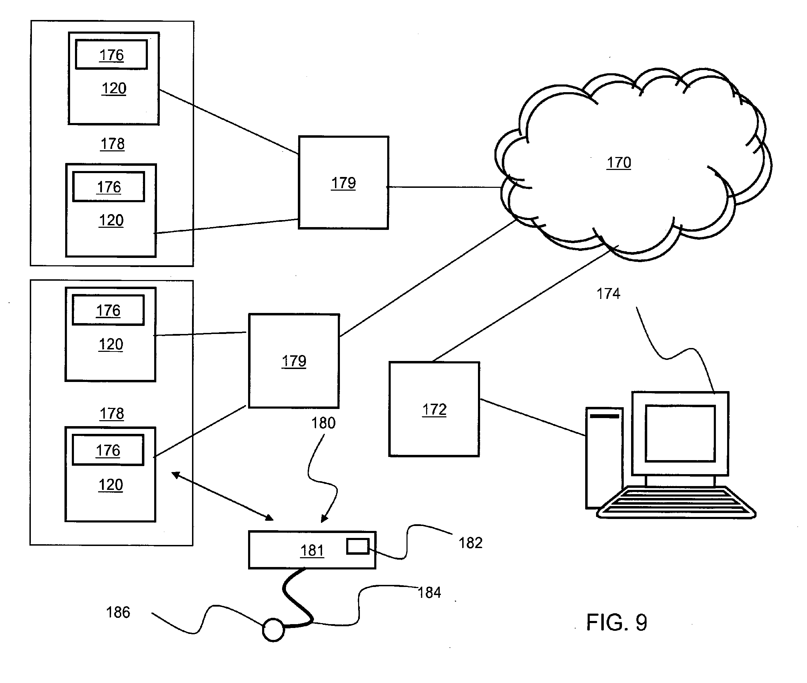

Operation of the device is as follows:
The cuff portion (181, see Fig 9) of a sphygmomanometer (180, fig. 9) is inserted into entrance 10 of passageway 1 (between the major and minor housing parts 7a, 7b) with rubber tubing 184 thereof facing towards the user projecting out of end opening 24. With the device 20 switched on, the presence of the cuff 181 is detected by sensor 56, and the controller, in accordance with its internal logic, signals the UV lamps 5 to switch on, and the electric motors 52 to drive. The rollers 2 rotate and move the cuff through the device towards exit 11 of the passageway 1. The controller 50 also signals the LED 6 to illuminate and indicate the device 20 is in operation. Of course, in other embodiments, the locations of entry and exit may be reversed, or be interchangeable.

The UV lamps 5 are adapted to prevent UV rays being scattered outside the unit. Once the cuff has passed through the device, the sensor 56 at the exit 11 detects this and the controller 50 signals the motors 52 and UV lamps 5 to deactivate.

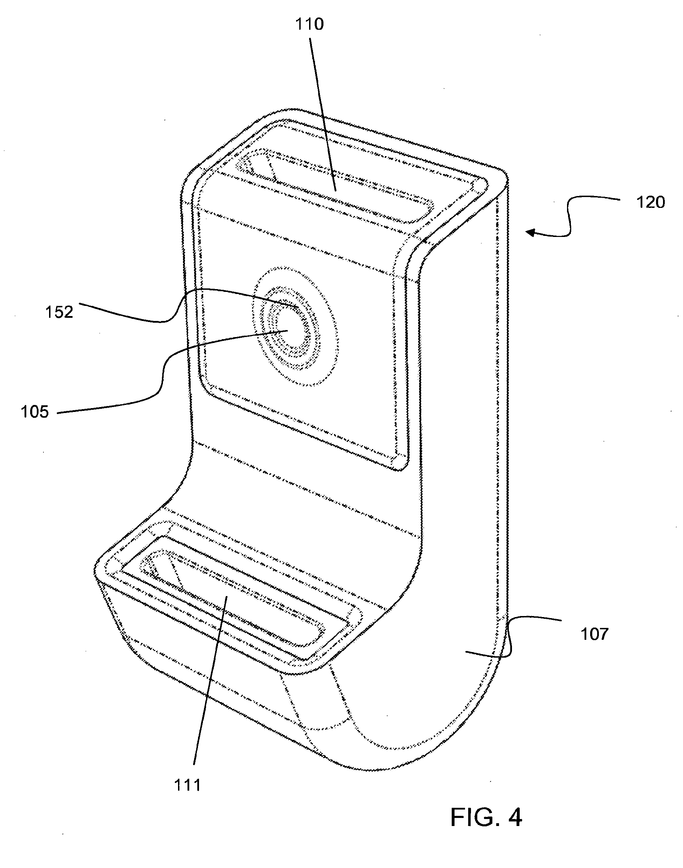

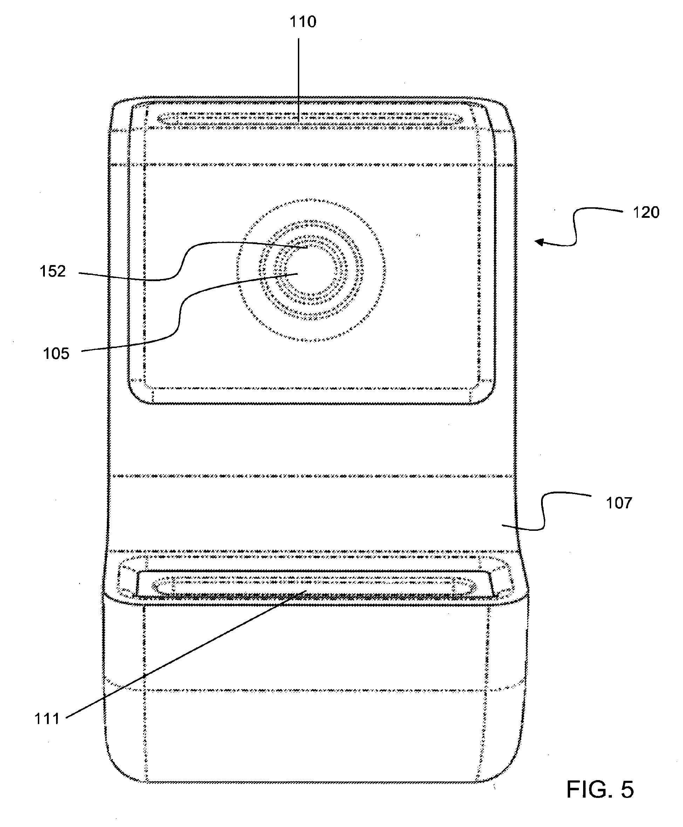

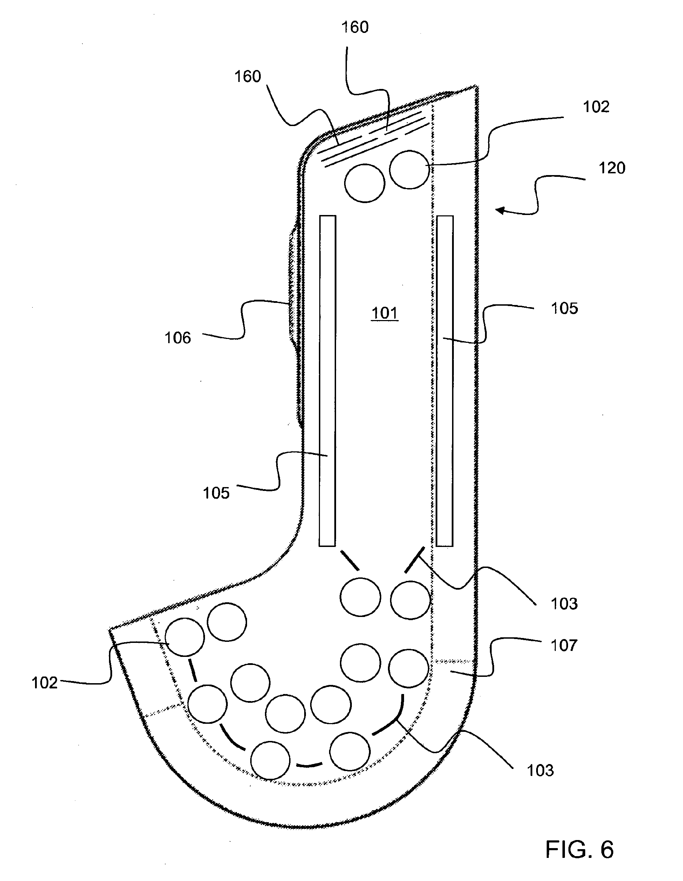

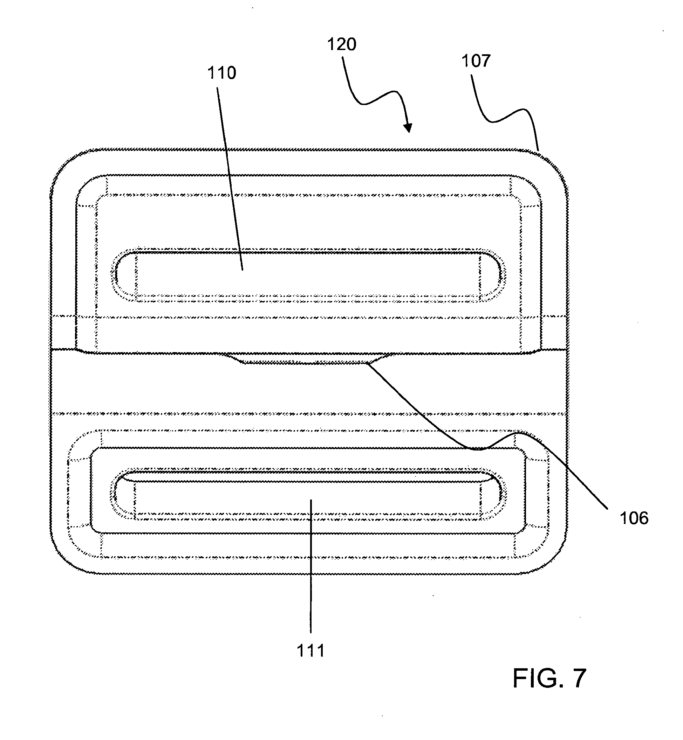

Referring now to the device 120 of the second embodiment of the present invention, as shown in Figures 4 to 7, like parts are illustrated by like numerals, with the addition of the prefix "1". Only those differences with the first embodiment are discussed in more detail below.

The device 120 operates using similar principles to device 20, but has a differing, predominantly vertical, path of a sphygmomanometer cuff 181 through the passageway 101, but at a lower section thereof, the passageway turns through an angle, in this case of approximately 140°, to present the cleaned sphygmomanometer cuff 181 to a user at the exit 111. It will be appreciated that this arrangement has a two-fold benefit. Firstly, it enables the sphygmomanometer cuff to be presented in a user-friendly, convenient location to the user, and secondly, it reduces the amount of UV light that may otherwise escape through the exit 111.

In addition, the device 120 comprises at entrance 110 a series of aligned pairs of flexible synthetic rubber fins 160, either side of the entrance, whose tips are adjacent or in mutual contact. In this embodiment, three layers of aligned pairs are provided, and the lengths of the pairs of sheets vary such that the tips are offset. This enables the sheets to act as a barrier to the escape of UV light, but at the same time the fins 160 may deflect to allow a sphygmomanometer cuff to pass through. Although not illustrated, a similar arrangement may be provided at the exit 111.

A pair of feed rollers 102 are provided below the fins 160 and at least one, preferably both, of each pair are driven by suitable motor(s) to feed a cuff into the device 120. At least one feed roller is resiliently mounted so as to be able to retract and accommodate cuffs of varying thickness. In other embodiments, the rollers may alternatively or additionally be flexible, so as to bend to accommodate cuffs.

In this embodiment, vertically arranged UV lamps 105 are positioned towards the front and rear of the housing 107 to define a passageway 101 through which a sphygmomanometer cuff is fed. As in the first embodiment, suitable reflectors (not shown) may be provided to ensure that substantially the entirety of the cuff is cleaned.

Further rollers 102, preferably provided in conjunction with guide surfaces 103, are arranged to turn the cuff through the required angle to feed it to the exit 111. It will be appreciated that the number of rollers may be varied according to the angle through which the cuff 181 is required to turn.

The front of the housing is provided with an on/off switch 152 and LED indicator 106 combined therewith.

The device 120 is further provided with a control system similar to that of the first embodiment, but without a breakaway sensor.

Operation of the device 120 is similar to that of the first embodiment. However, in this embodiment, the display and bulb 186 and inflation tube 184 are first removed from the cuff before insertion into the entrance 110.

Additionally, the controller may be programmed such that the rollers 102 at the exit 111 retain the cuff 181 until manually removed by a user (i.e. to prevent it falling to the floor).

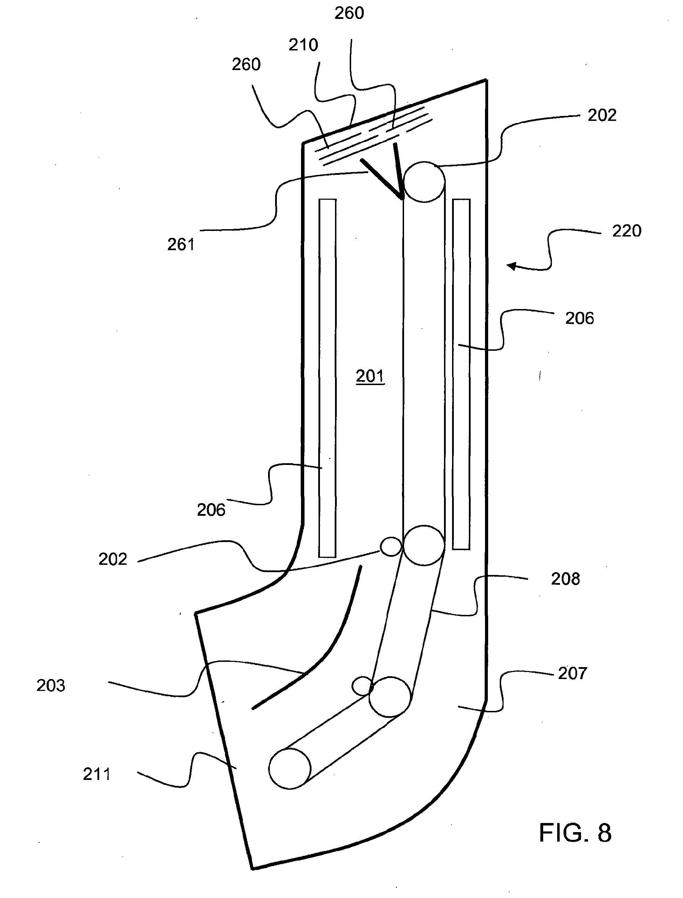

Fig. 8 illustrates a device 220 according to a third embodiment of the present invention. Like parts are denoted by like numerals, but with the addition of the prefix "2".

The device 220 replaces the opposed roller mechanism of the first embodiment with gripping mechanism, e.g. a jaw mechanism comprising a jaw 261 mounted on a belt 208 guided by pulleys 202. The jaw 261 is configured to grab the leading edge of a sphygmomanometer cuff and then pull the cuff through the passageway at a suitable speed.

The passageway 201 of this embodiment turns through a lesser angle of approximately 60° and is defined on its frontal face by a guide surface 203.

Upon reaching the exit 211, the jaw holds the sphygmomanometer cuff until it is manually removed. The jaw is subsequently returned to its start position proximate the entrance 210 by a suitably programmed controller.

Thus, the device is simple, convenient and quick to operate, meaning that it is more likely that healthcare personnel will adhere to a cleaning regime, and sphygmomanometer cuffs cleaned with the device are less likely to harbour harmful bacteria. The feed mechanisms used enable sphygmomanometer cuffs of variable and uneven thickness to be cleaned with a single device.

It will be understood that numerous changes may be made within the scope of the present invention. In one variant of the device, the controller 50 is programmed to log use of the device and signal flashing of LED 6, 106 after a user defined period of use to indicate that the device itself requires internal cleaning. This internal cleaning may be performed with a specially designed swab, pad or device, preferably soaked in alcohol gel, that passes through the unit in the same manner as a sphygmomanometer cuff. In this variant of the device, once the device has been cleaned a reset button may be pressed to deactivate the flashing of LED 6, 106.

The device may be further configured to have two modes of operation, a standard quick clean mode for cleaning after each use of the of a sphygmomanometer cuff, and a second slower "deep clean" mode for enhanced cleaning of a sphygmomanometer cuff after a certain period of time, e.g. a week, or certain number of uses, e.g. 10. A further switch may be provided on the device to manually select the mode.

In a further variant, the device may be provided with a slot running down one side thereof such that the inflation tube 184 need not be detached from the cuff 181, and may be carried, protruding therethrough. The slot may have one or more fins provided, similar to those at the entrance to minimise the escape of UV light. A separate runner may be provided to drive the inflation tube along with the cuff.

The device may also have a curved passageway at its entrance to act as a means of inhibiting the escape of UV light.

Although the generally upright nature of the devices of the second and third embodiments assists with cooling of the devices through convection, additional cooling may be provided by providing additional vents or forced cooling from a fan or the like.

The devices of the second and third embodiments may also be provided with a breakaway mechanism to enable safe opening in the event of unwanted inflation of a cuff in the device. In addition the devices may be provided with diagnostic functions to ensure that the UV sources remain functional and do not emit harmful levels of ozone, e.g. by timing cumulative usage of the device and/or providing an ozone sensor and suitable warning mechanism. The devices may also be provided with means for sensing and overcoming jams - e.g. by reversing the rollers or clip, or by making the device openable for a user to release the cuff inside. Of course, in this event, controller 50 would automatically deactivate UV lamps 5, 105, 205.

In a still further variant, the inflation tube 184 may be provided with a suitable releasable connector, and the device 120, 220 with a mechanism to automatically release the inflation tube, if the cuff 181 is inserted with it still connected, In other variants, the user is expected to remove the inflation tube 184, and the device may be configured to reject cuffs 181 if the inflation tube 184 is still connected when inserted.

The device may be adapted for use with alternative or additional further means of cleaning or disinfection, including ultrasonic or silver based techniques.

In a variant of the device, it additionally comprises a hand-held cleaning unit removably mounted on the housing and configured to clean further portions of a sphygmomanometer, such as a cord thereof. The hand-held device may be rechargeable and therefore cordless, or be powered from the device, in which case it may be connected thereto by a cord. The housing may be provided with a docking station to enable the hand held unit to be mounted on and optionally electrically charged from the housing.

In a further embodiment illustrated in Fig 9. each sphygmomanometer 180 is tagged with, for example an RFID tag 182, (or in other embodiments a barcode, QR code, or other suitable machine readable identifier) and the device is provided with a corresponding reader 176, e.g. a short range RFID reader (not shown).

In a basic variant, the controller associated with an individual device 120 may log the number of quick cleans, or time elapsed since the last deep clean, and automatically determine whether a deep clean or quick clean is needed.

However, in larger organisations, such as a large hospital, or across a series of primary care clinics, an individual cuff 181 may be used multiple devices 20, 120, 220 in multiple wards or clinics 178 at the same or in different locations. Therefore in a preferred embodiment for such situations each device 20, 120, 220 is provided with a mechanism to transmit data regarding cuff cleaning events to a remote location.

In the preferred embodiment the data transmission mechanism is an RF transmitter functional at a frequency (e.g. UHF) and power level whereby interference with other equipment will not occur, which transmits to a suitable receiver within range thereof.

In a particularly preferred embodiment, the transmitter transmits packet data using a recognised protocol such as 802.11b, 802.11a, 802.11g, 802.11n, wired Ethernet, Zigbee, DECT, SMS, GPRS, UMTS, WiMAX, LTE or the like to a suitable gateway 179 such as an internet wireless router or a mobile telephone base station connected to the internet or an alternative computer network 170. A computer database 172 connected to the network is thereby able to hold data on individual cuff 180 cleaning events including the unique identifier of the cuff to be cleaned and the location and/or a unique identifier of the device where it is cleaned.

This database may therefore be used to determine if sufficient standard cleaning events have occurred to merit a deeper clean, irrespective of the devices 20, 120, 220 used for each clean, and indeed whether a particular cuff 181 is one that is permitted to be used with a particular device. The database 172 may therefore communicate back to devices 20, 120, 220 whether a standard clean is required, a deeper clean, or that the device will not accept the insertion of that cuff 181 to be cleaned. To this end, the device may be provided with a door at the entrance that only opens in the event that an approved cuff,, as opposed to a non-approved cuff or another object entirely, is presented for cleaning.

Additionally the database 172 enables the cleaning of cuffs 181 to be remotely audited at a computer, tablet, smartphone or other network-connected device 174. The database may be accessible via a standard internet browser using HTML or other standard web pages. Medical personnel may therefore be readily informed when they are not adhering to protocol laid down by the organisation in which the devices are installed. Medical personnel may be sent messages regarding their usage or non-compliance via e.g. email or SMS.

The database may also be used as a way of monitoring the function of the devices and being able log errors in the function of the devices.

In a further variant, the tag 182 itself on the sphygmomanometer may log the number of quick cleans in its own memory, and supply this information to the device 20, 120, 220. Then, even if multiple devices are used around a hospital, the correct cleaning regimen may be maintained without the need for a central database. However, the auditing function in this system may require a periodic download of data from a cuff at predetermined location(s) in order to compile the information into a database.

In a still further variant, if an electronic sphygmomanometer is used, this may itself be configured to communicate with the devices of the present invention, so that operation of the sphygmomanometer is prevented unless it has been cleaned since its last use, or a certain threshold number of uses or time since last clean has elapsed. The tag 182 on the cuff 181 may enable this communication. However, it may be desirable to build an override function into the sphygmomanometer to enable usage in emergencies. The usage of this override function may also be logged and reported to prevent it being abused.

Devices may also be provided with a geo-location function (e.g. via GPS, cell tower and/or IP address), such that stolen devices may be tracked and/or shut down from operation.

## Claims

1. A device (20, 120, 220) to clean and/or disinfect either or both surfaces of a sphygmomanometer cuff, the device comprising a source of cleaning and/or disinfection (5, 105, 205) located so as to act on a sphygmomanometer cuff; **characterised in that**
an entrance (10, 110, 210) through which a sphygmomanometer cuff may be introduced into a passageway (1, 101, 201) and
an exit (11, 111, 211) from the passageway is provided, said source of cleaning and/or disinfection (5, 105, 205) located within the passageway; **in that** a drive mechanism (2, 102, 202) is provided to feed said cuff through the passageway and **in that** the passageway comprises a curved portion.

2. A device as claimed in Claim 1 configured to clean and/or disinfect both surfaces of a sphygmomanometer cuff simultaneously; preferably further comprising a source of cleaning and/or disinfection (5, 105, 205) on first and second sides of the passageway to clean and/or disinfects both surfaces of a sphygmomanometer cuff simultaneously.

3. A device according to claim 1 or claim 2 wherein the curved portion is provided proximate the exit (11, 111, 211); preferably wherein the curved portion diverts the passageway through an angle of at least 30°.

4. A device according to any preceding claim wherein the passageway comprises a substantially straight portion housing the source of cleaning and/or disinfection.

5. A device as claimed in any preceding Claim, wherein the source of cleaning and/or disinfection is ultraviolet light; preferably further comprising a scattering arrangement to scatter the light and reach maximal coverage of a sphygmomanometer cuff surface; more preferably wherein the scattering arrangement comprises a multi-faceted mirror.

6. A device according to any preceding claim wherein the drive mechanism comprises a gripping mechanism (2, 102, 202) to engage a cuff.

7. A device according to any preceding Claim wherein the drive mechanism comprises at least one feed roller (2, 102); preferably wherein the feed roller is flexible to accommodate uneven thicknesses of sphygmomanometer cuff.

8. A device according to any preceding claim comprising an adjustment mechanism for the passageway to permit the passageway to adjust to accommodate sphygmomanometer cuffs of differing overall thickness.

9. A device according to any preceding claim further comprising a control mechanism (20) to detect the presence of a sphygmomanometer cuff within the passageway, and only permit the source of cleaning and/or disinfection to be activated with a sphygmomanometer cuff present within the passageway.

10. A device according to any one of claims 1 to 9 further comprising an ejection mechanism to eject a sphygmomanometer cuff in the event of an accidental inflation of a sphygmomanometer cuff within the device so as to minimise the risk of damage occurring.

11. A device as claimed in any preceding Claim further comprising a means of attachment (9) to trolleys and/or other portable equipment.

12. A device according to any preceding claim further comprising an arrangement (160, 260) located at at least one of the entry and exit to inhibit the escape of UV light therefrom.

13. A device according to claim 12 wherein the arrangement comprises at least one pair of opposed flexible fins (160, 260); preferably wherein a second pair of fins having tips thereof offset from the first are provided.

14. A device as claimed in any preceding Claim further comprising a means of indicating when servicing or cleaning is required.

15. A method of cleaning or disinfecting a sphygmomanometer cuff utilising a device (20, 120, 220) according to any preceding claim, comprising a step of feeding the sphygmomanometer cuff through the entrance (10, 110, 210) of the device into the passageway (1, 101, 201).

## Patentansprüche

1. Eine Vorrichtung (20, 120, 220) zum Reinigen und/oder Desinfizieren einer oder beider Oberflächen einer Blutdruckmessgerätmanschette, wobei die Vorrichtung eine Reinigungs- und/oder Desinfektionsquelle (5, 105, 205) aufweist, welche so angeordnet ist, dass sie auf die Blutdruckmessgerätmanschette wirkt, **dadurch gekennzeichnet, dass**
ein Einlass (10, 110, 210), durch welchen die Blutdruckmessgerätmanschette in einen Durchlass (1, 101, 201) einbringbar ist und ein Auslass (11, 111, 211) aus dem Durchlass vorgesehen ist,
wobei die Reinigungs- und/oder Desinfektionsquelle (5, 105, 205) innerhalb des Durchlasses angeordnet ist; und
dass ein Antriebsmechanismus (2, 102, 202) vorgesehen ist, um die Manschette durch den Durchlass zu fördern und dass der Durchlass einen gekrümmten Abschnitt aufweist.

2. Eine Vorrichtung nach Anspruch 1, ausgelegt zur Reinigung und/oder Desinfektion gleichzeitig beider Oberflächen einer Blutdruckmessgerätmanschette, bevorzugt weiterhin aufweisend eine Reinigungs- und/oder Desinfektionsquelle (5, 105, 205) an ersten und zweiten Seiten des Durchlasses zum Reinigen und/oder Desinfizieren gleichzeitig beider Oberflächen der Blutdruckmessgerätmanschette.

3. Eine Vorrichtung nach Anspruch 1 oder 2, wobei der gekrümmte Abschnitt den Durchlass um einen Winkel von wenigstens 30° ablenkt.

4. Eine Vorrichtung nach einem der vorhergehenden Ansprüche, wobei der Durchlass einen im wesentlichen geraden Abschnitt aufweist, der die Reinigungs- und/oder Desinfektionsquelle aufnimmt.

5. Eine Vorrichtung nach einem der vorhergehenden Ansprüche, wobei die Reinigungs- und/oder Desinfektionsquelle ultraviolettes Licht ist, bevorzugt weiterhin aufweisend eine Streuanordnung zum Streuen des Lichts und zum Erreichen einer maximalen Abdeckung einer Oberfläche der Blutdruckmessgerätmanschette, wobei besonders bevorzugt die Streuanordnung einen Vielfachfacettenspiegel aufweist.

6. Eine Vorrichtung einem der vorhergehenden Ansprüche, wobei der Antriebsmechanismus einen Greifmechanismus (2, 102, 202) für einen Eingriff mit der Manschette aufweist.

7. Eine Vorrichtung nach einem der vorhergehenden Ansprüche, wobei der Antriebsmechanismus wenigstens eine Förderrolle (2, 102) aufweist, wobei bevorzugt die Förderrolle flexibel ist, um sich Dickenunebenheiten der Blutdruckmessgerätmanschette anzupassen.

8. Eine Vorrichtung nach einem der vorhergehenden Ansprüche, aufweisend einen Einstellmechanismus für den Durchlass, um eine Einstellung des Durchlasses zur Aufnahme von Blutdruckmessgerätmanschetten unterschiedlicher Gesamtdicken zu ermöglichen.

9. Eine Vorrichtung nach einem der vorhergehenden Ansprüche, weiterhin aufweisend einen Steuermechanismus (20) zur Erkennung des Vorhandenseins einer Blutdruckmessgerätmanschette innerhalb des Durchlasses und um eine Aktivierung der Reinigungs- und/oder Desinfektionsquelle nur dann zu erlauben, wenn sich eine Blutdruckmessgerätmanschette innerhalb des Durchlasses befindet.

10. Eine Vorrichtung nach einem der Ansprüche 1 bis 9, weiterhin aufweisend einen Auswurfmechanismus zum Auswerfen der Blutdruckmessgerätmanschette im Fall eines unbeabsichtigten Aufblasens der Blutdruckmessgerätmanschette innerhalb der Vorrichtung, um das Auftreten von Schäden zu minimieren.

11. Eine Vorrichtung nach einem der vorhergehenden Ansprüche, weiterhin aufweisend Anbringmittel (9) für Handwägen und/oder anderes tragbares Gerät.

12. Eine Vorrichtung nach einem der vorhergehenden Ansprüche, weiterhin aufweisend eine Anordnung (160, 260), welche an wenigstens entweder dem Einlass oder dem Auslass liegt, um den Austritt von UV-Licht hieraus zu unterbinden.

13. Eine Vorrichtung nach Anspruch 12, wobei die Anordnung wenigstens ein paar von gegenüberliegenden flexiblen Flügeln (160, 260) aufweist, wobei bevorzugt ein zweites Paar von Flügeln vorgesehen ist, deren Spitzen gegenüber denjenigen des ersten Paares versetzt sind.

14. Eine Vorrichtung nach einem der vorhergehenden Ansprüche, weiterhin aufweisend Mittel zur Anzeige, ob Wartung oder Reinigung notwendig ist.

15. Ein Verfahren zum Reinigen oder Desinfizieren einer Blutdruckmessgerätmanschette unter Verwendung einer Vorrichtung (20, 120, 220) nach einem der vorhergehenden Ansprüche, aufweisend einen Schritt des Einführens der Blutdruckmessgerätmanschette durch den Einlass (10, 110, 210) der Vorrichtung in den Durchlass (1, 101, 201).

## Revendications

1. Dispositif (20, 120, 220) pour nettoyer et/ou pour désinfecter l'une des deux ou les deux surfaces d'un brassard sphygmomanomètre, le dispositif comprenant une source de nettoyage et/ou de désinfection (5, 105, 205) située afin d'agir sur un brassard sphygmomanomètre ; **caractérisé en ce que** :
une entrée (10, 110, 210), à travers laquelle un brassard sphygmomanomètre peut être introduit dans une voie de passage (1, 101, 201) et
une sortie (11, 111, 211) de la voie de passage sont prévues,
ladite source de nettoyage et/ou de désinfection (5, 105, 205) étant située à l'intérieur de la voie de passage ; **en ce qu'**un mécanisme d'entraînement (2, 102, 202) est prévu pour faire avancer ledit brassard à travers la voie de passage et **en ce que** la voie de passage comprend une partie incurvée.

2. Dispositif selon la revendication 1, configuré pour nettoyer et/ou pour désinfecter les deux surfaces d'un brassard sphygmomanomètre simultanément ; de préférence comprenant en outre une source de nettoyage et/ou de désinfection (5, 105, 205) sur des premier et second côtés de la voie de passage pour nettoyer et/ou pour désinfecter les deux surfaces d'un brassard sphygmomanomètre simultanément.

3. Dispositif selon la revendication 1 ou la revendication 2, dans lequel la partie incurvée est prévue à proximité de la sortie (11, 111, 211) ; de préférence dans lequel la partie incurvée dévie la voie de passage selon un angle d'au moins 30°.

4. Dispositif selon l'une quelconque des revendications précédentes, dans lequel la voie de passage comprend une partie sensiblement droite logeant la source de nettoyage et/ou de désinfection.

5. Dispositif selon l'une quelconque des revendications précédentes, dans lequel la source de nettoyage et/ou de désinfection est de la lumière ultraviolette ; de préférence comprenant en outre un agencement de diffusion pour diffuser la lumière et atteindre une couverture maximale d'une surface de brassard sphygmomanomètre ; mieux encore dans lequel l'agencement de diffusion comprend un miroir multi-facette.

6. Dispositif selon l'une quelconque des revendications précédentes, dans lequel le mécanisme d'entraînement comprend un mécanisme de préhension (2, 102, 202) pour entrer en prise avec un brassard.

7. Dispositif selon l'une quelconque des revendications précédentes, dans lequel le mécanisme d'entraînement comprend au moins un rouleau d'avance (2, 102) ; de préférence dans lequel le rouleau d'avance est flexible pour s'adapter à des épaisseurs irrégulières de brassard sphygmomanomètre.

8. Dispositif selon l'une quelconque des revendications précédentes, comprenant un mécanisme d'ajustement pour la voie de passage pour permettre à la voie de passage de s'ajuster pour loger des brassards sphygmomanomètres d'épaisseurs totales différentes.

9. Dispositif selon l'une quelconque des revendications précédentes, comprenant en outre un mécanisme de commande (20) pour détecter la présence d'un brassard sphygmomanomètre à l'intérieur de la voie de passage, et seulement permettre à la source de nettoyage et/ou de désinfection d'être activée avec un brassard sphygmomanomètre présent à l'intérieur de la voie de passage.

10. Dispositif selon l'une quelconque des revendications 1 à 9, comprenant en outre un mécanisme d'éjection pour éjecter un brassard sphygmomanomètre en cas de gonflement accidentel d'un brassard sphygmomanomètre à l'intérieur du dispositif afin de minimiser le risque de dommage.

11. Dispositif selon l'une quelconque des revendications précédentes, comprenant en outre un moyen de fixation (9) à des chariots et/ou à d'autres équipements portatifs.

12. Dispositif selon l'une quelconque des revendications précédentes, comprenant en outre un agencement (160, 260) situé à au moins une parmi l'entrée et la sortie pour empêcher la sortie de lumière UV de celle-ci.

13. Dispositif selon la revendication 12, dans lequel l'agencement comprend au moins une paire d'ailettes flexibles opposées (160, 260) ; de préférence dans lequel une seconde paire d'ailettes, dont des embouts sont décalés par rapport à ceux de la première, sont prévues.

14. Dispositif selon l'une quelconque des revendications précédentes, comprenant en outre un moyen d'indiquer lorsqu'un entretien ou un nettoyage est requis.

15. Procédé de nettoyage ou de désinfection d'un brassard sphygmomanomètre en utilisant un dispositif (20, 120, 220) selon l'une quelconque des revendications précédentes, comprenant une étape de l'avance du brassard sphygmomanomètre à travers l'entrée (10, 110, 210) du dispositif dans la voie de passage (1, 101, 201).
